# EUROPEAN PATENT SPECIFICATION

(11) **EP 2 790 819 B1**
(45) Date of publication and mention of the grant of the patent: **17.01.2018**
(21) Application number: 12818845.5
(22) Date of filing: 14.12.2012
(51) Int. Cl.: B01D 65/00, A61M 1/36

(54) **BLOOD FILTER WITH ATTACHMENT ELEMENT**
BLUTFILTER MIT BEFESTIGUNGSELEMENT
FILTRE AVEC ÉLÉMENT D'ATTACHEMENT

(30) Priority: 16.12.2011 US 201161576492 P
(43) Date of publication of application: 22.10.2014
(73) Proprietor: Terumo BCT, Inc., Lakewood, CO 80215 (US)
(72) Inventor: GIBBS, Bruce W., Arvada, Colorado 80004 (US); DOLECEK, Victor D., Englewood, Colorado 80111 (US)
(74) Representative: J A Kemp
(86) International application number: PCT/US2012/069726
(87) International publication number: WO 2013/090707

(56) References cited:
- WO-A1-01/56679
- FR-A1- 2 821 762
- US-A- 4 976 707
- US-A- 5 695 489
- US-A1- 2005 133 439

## Description

Filter media for filtering blood components is frequently encased in a hard plastic housing. Alternatively, the housing for the layered or foam filter media may be made of soft or flexible thermoplastic resin sheets resulting in what may be called a soft filter. It is to such a soft filter or soft housing filter that embodiments of the instant invention are directed.

One of the challenges of manufacturing a soft housing filter is the attachment between the soft thermoplastic housing sheets and the filter media layers or filter or media pack itself. Some of the challenges relate to the inability or difficulty in bonding diverse or incompatible, (for bonding purposes), materials, e.g., plastics. A further challenge may be the bonding of filter media (which may be sheets of porous or fibrous material) to inlet and outlet tubes of the filter. Past solutions have included sealing the filter media layers or media pack by heat or thermal welding directly between the soft thermoplastic sheets on all edges of the soft housing sides. Such sealing would then bond the filter layers to the inlet and outlet tubes. This solution may not solve the binding compatibility issues depending on the materials utilized.

Another past solution includes providing a frame all around the edges of the filter media pack and sealing such edges of the media pack to the frame. The frame is then sealed, such as by heat or thermal welding, between the soft thermoplastic housing sheets and to the inlet and outlet tubes. Such frame may present extra construction costs as well as extra construction steps. The frame may also reduce the overall effective filtering surface within the housing in that it may reduce the overall surface available for filtering. Prior art filters with flexible outer housing and tube connections for filtering blood or blood components are disclosed in FR 2821762, US 4,976,707, US 5,695,489 or WO01/56679. Filtering for removing leukocytes in blood components can be achieved through using a gravity drain procedure wherein the fluid to be filtered is allowed to drain by gravity from a container, through the filter inlet, through the filter media from one side to the other or through layers of the filter media, and out through the filter outlet.

Alternatively, in some systems the fluid to be filtered may be pushed through the filter using a pump or an expressing device. The fluid to be filtered can also be pushed through the filter using a force, such as centrifugal force, to move the fluid.

The invention is defined in the claims.

Embodiments of the present invention are directed to a filter. The filter includes an inlet tube; an outlet tube; an outer housing attached to the inlet tube and outlet tube; a first attachment element attached to one of the inlet or outlets tubes; and filter media in the outer housing, wherein the filter media may be attached to the first attachment element. In one embodiment, the first attachment element may attach the filter media to the outlet tube but not the inlet tube. The first attachment element includes a first wing with a tip and a second wing with a tip. Such attachment element may be between the filter media and one of the inlet and outlet tubes. A second attachment element may be attached to the same one of the inlet and outlet tubes as the first attachment element. The second attachment element may include a third wing with a tip and a fourth wing with a tip and the second filter media may be further attached to the third and fourth wings. Filter media may be attached to the side of first, second, third and fourth wings, each of the tips of the wings may contact the outer housing to direct the blood or blood component through the filter media. The filter media may comprise layers, and the layers may be sealed together at a second end opposite the first end of the filter media. At least one of the inlet and outlet tubes may extend between the first and second attachment elements and between the filter media.

A method of manufacturing a filter is also claimed.

The invention will be further described, by way of example, with reference to the accompanying drawings, in which:
FIG. 1 illustrates a soft housing filter according to one embodiment.
FIG. 2 illustrates the soft housing filter of FIG. 1 with a portion of an outer housing removed.
FIG. 3 is a cross-sectional view of the filter illustrated in FIG. 2.
FIG. 4 is a perspective view of an embodiment of an attachment element.
FIG. 5 is a perspective view of another embodiment of an attachment element.
FIG. 6 is a bottom perspective view of another embodiment of an attachment element.
FIG. 7 is a flow chart illustrating a method of manufacturing a filter according to embodiments.

For purposes of illustration, embodiments of the present invention will be described with respect to a blood filter, for example a leukoreduction filter, for removing leukocytes from blood components or blood products. The embodiments of a leukoreduction filter include media encased in housing. It is noted that although the description may refer to leukoreduction, the present invention is not limited to this specific use, and may in other embodiments, be used to filter other fluid, of blood.

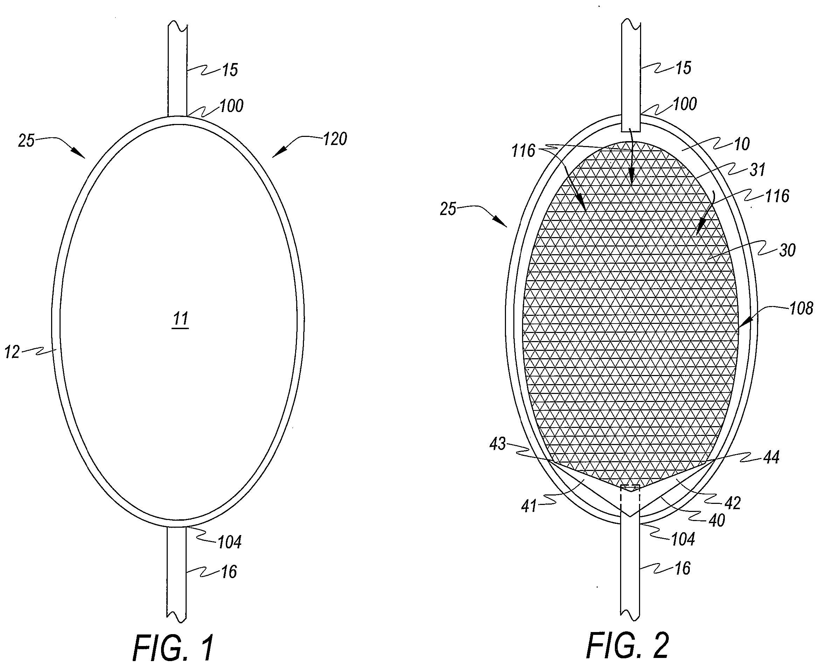

The media for filtering may be formed of any suitable material including, without limitation, woven material, non-woven material, or fibers oriented in specific ways. Alternatively, a porous foam material can be used. Media may be stacked in layers to form a pack or packs and optionally and selectively calendared to form different shapes, and in some embodiments shapes that correspond to the shapes of the filter housing. In some embodiments, the shape may be disk shaped. The edges of the layers of media may be heat or thermally welded together or attached using some other bonding techniques, including without limitation, solvent welding, heat welding, high frequency welding (e.g., RF (radio frequency) welding or ultrasonic welding), and/or laser welding. FIG 2 illustrates bonding of layers of media at an edge 31. In embodiments, synthetic, semi-synthetic or natural fibers may be used for the media. For example, polyester fibers such as polyethylene terephthalate, nylon, polypropylene, polyacrylnitrile and the like may be used. The media in embodiments may receive treatment to enhance wet-ability or filter functionality, e.g., chemical, heat, or other types of treatments.

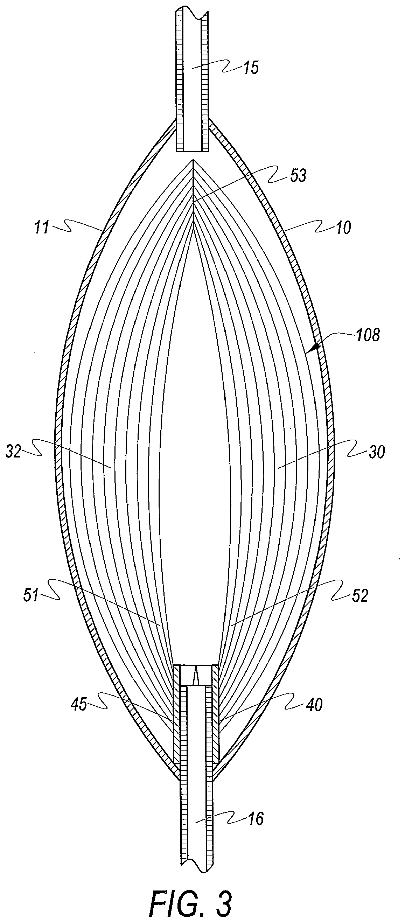

FIG. 1 shows the front of an embodiment of a soft housing filter 25. For the filter 25, a first sheet 11 is sealed or bonded by heat or thermal sealing or other known sealing methods to a second sheet 10 around peripheral edge 12 to form an outer housing 120 made of soft material within which filter media is positioned (see FIG. 3). Sheets 10 and 11 are flexible in some embodiments and may in some embodiments be made of thermoplastic resin. Sheets 10 and 11 may optionally be textured to provide an uneven surface or have a surface treatment to prevent filter media packs 30 and 32 (described below) from adhering to such sheets 10 and 11.

A port 100 in the outer housing 120 is shown in FIG. 1 with tube 15 positioned in the port 100. The tube 15 attached to and between sheets 10 and 11. FIG. 1 also shows a second port 104 with tube 16 positioned in the port 104. Tube 16 is attached to and between the sheets 10 and 11. The ports 100 and 104, with tubes 15 and 16, are designated by the direction of flow and can serve as either inlets or outlets for the filter 25. For example, arrows 116 in FIG. 2 illustrate the flow of fluid when tube 15 is an inlet tube and fluid flows through the filter media from inlet tube 15 to outlet tube 16. In embodiments where tube 16 is an inlet tube, filter 25 would be turned 180 degrees (from the illustration in FIG. 2) when used to filter fluid and the flow of fluid through the filter media would also be approximately 180 degrees from the flow shown in FIG. 2.

Filter media material for filtration is shown in FIG. 2 positioned within the outer housing 120 created by sheets 10 and 11. The filter media material is also shown in FIG. 3. The filter media may be in the form of packs 30 and 32 which include multiple layers of filter media and which may include calendared layers. In the example filter, the layers of the two filter packs 30 and 32 are welded together around their peripheral edges such as edge 31 for pack 30 as shown in FIG. 2. The filter media or packs 30 and 32 are attached to tube 16 by barriers or attachment elements 40 and 45 to create a filter assembly 108, as described below.

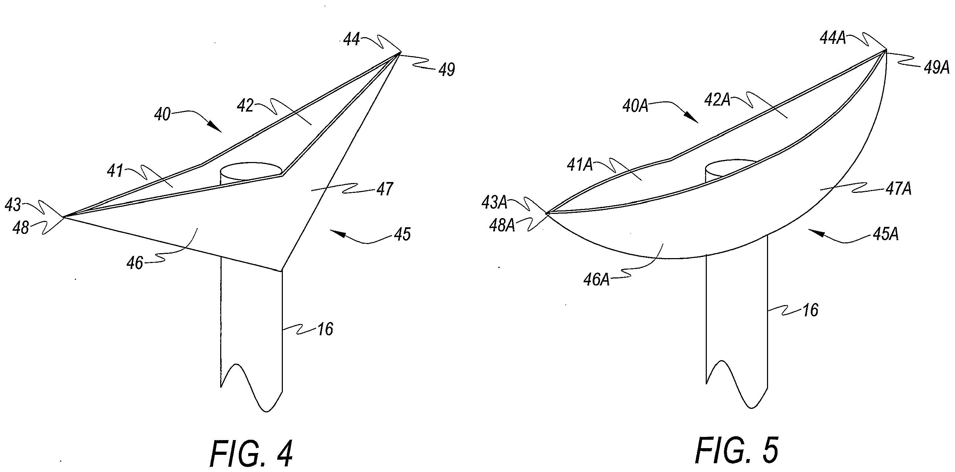

Tube 15 and tube 16 may be formed of soft or hard plastic tubing material a nonlimiting example including, polyvinyl chloride (PVC). First attachment element 40 (as shown in FIG. 2 and FIG. 4), in embodiments includes first wing 41 and second wing 42. FIG. 2 shows first attachment element 40 and the first of filter media layers or pack 30 attached thereto. Second attachment element 45 is shown in FIG. 3 with the second of filter media layers or pack 32 adhered thereto. Wings 41 and 42 of the first barrier 40 and the third and fourth wings 46 and 47 of second barrier 45 (FIG. 4) assure the fluid to be filtered passes through the filter media packs 30 and 32. As shown in FIG. 2, first barrier or attachment element 40 and first and second barrier tips 43 and 44 extend toward the soft side wall sheet 10 and 11 forming the outer housing 120 of filter 25. Second barrier or attachment element 45 has similar third and fourth barrier tips 48, 49 which also extend to the sheets 10 and 11. When tube 16 is attached to attachment elements 40 and 45, tube 16 extends between the attachment elements.

Attachment elements or barriers 40 and 45 may also be constructed as a one piece element, prior to attachment to the filter media and the tube 16 as described below, with tip 43 attached to tip 48 and tip 44 attached to tip 49 by heat sealing, solvent bonding or other known sealing methods. The attachment elements may be made of suitable plastic material such as thermoplastic material. The attachment elements or barriers 40 and 45 may also be molded as a one piece construction. The combination of filter media packs 30 and 32, and the attachment elements 40 and 45 create a filter assembly 108 which is positioned within the outer housing.

The barriers or attachment elements 40 and 45 of the filter assembly 108 are bonded by solvent or thermal bonding or other known methods to the tube 16 on either side of the tube 16 in an opposite relationship. As shown in FIG. 3, one side or inner layer 52 of the filter media or pack 30 is attached by heat, solvent or thermal bonding to first barrier or attachment element 40 along the wings 41 and 42. The other layers (if fibrous multi-layer media is used) are attached to the side or inner layer 52. Similarly, one side or inner layer 51 of filter media pack 32 is attached to second barrier or attachment element 45 along its wings by suitable bonding technique with other layers attached or welded to the side or inner layer 51. Alternatively, if appropriately sized, multiple layers of each of the filter media pack 30 and 32 of the filter 25, may be attached to the barriers or attachment elements 40 and 45. As shown in the embodiment of FIG. 3, the filter media layers of packs 30 and 32 are not directly attached to soft housing sheets 11 and 10 but are attached to barrier or attachment elements 40 and 45. Similarly the media layers or packs 30 and 32 are not directly attached to the tube 16. The tube 16, however, is attached to the barrier or attachment elements 40 and 45.

The barrier or attachment elements 40 and 45 prevent the fluid to be filtered from by-passing one of the filter media packs 30 or 32. Filter media packs 30 and 32 are sealed together at the top at 53 by welding so that fluid must go through filter media 30 or 32 to reach tube 16. In embodiments, the filter media packs 30 and 32 are not attached to the tube 15. Similarly the attachment of the filter packs 30 and 32 through the inner layer, and barrier or attachment element 40 or 45 prevents fluid from flowing directly to the tube 16 and bypassing the filter pack.

The barrier or attachment elements 40 and 45 are shown connecting the filter media packs 30 and 32 to the tube 16. In embodiments, the barrier or attachment elements 40 and 45 may optionally be used on the tube 15, with the filter packs 30 and 32 sealed to each other on the side of the filter 25 near the tube 16 but not attached thereto. In this instance, flow would be from the tube 15 between the two filter media packs 30 and 32, through the media of such packs and then to the tube 16. Tubes 15 and 16 may in embodiments be configured to serve as either inlet or outlet tubes of filter 25, allowing fluid to flow into and out of filter 25.

FIG. 5 illustrates another design of attachment elements. As shown in FIG. 5, the attachment elements 40A and 45A, and wings (41A, 42A, 46A, and 47A) on the attachment elements, may have a different shape. The wings shown in FIG. 5 are larger and have a shallower angle, than the wings shown in FIGS. 1-4. In other embodiments, the tips (43A, 44A, 48A, and 49A) of the wings may be rounded, end in an edge instead of a tip, or have other designs or shapes. FIG. 5 is shown to illustrate other designs that are contemplated within the scope of the present invention. Other attachment elements with different wings or other structural features will be apparent to those of skill in the art and are within the scope of the present invention.

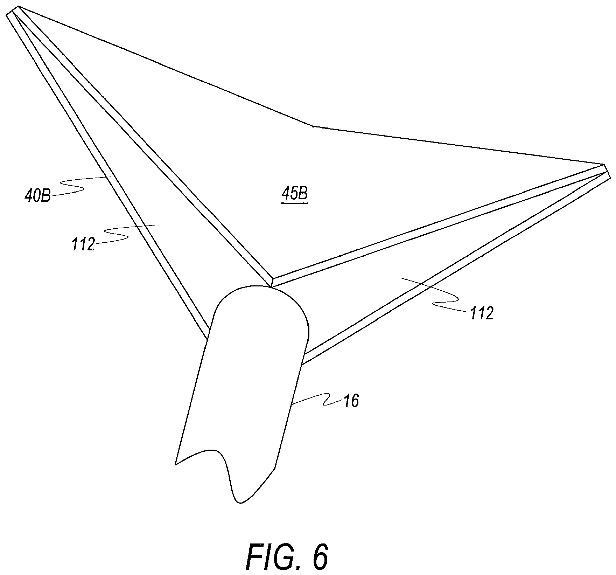

FIG. 6 shows a bottom perspective view of attachment elements that are consistent with another embodiment of the present invention. As shown in FIG. 6, some embodiments may include connecting material 112 that connects attachment elements 40B and 45B. As noted above, the tips of attachment elements, e.g., 40B and 45B may be connected; connecting material 112 further connects attachment elements 40B and 45B. The connecting material 112 may be angled to serve as a funnel that further funnels filtered fluid to tube 16. The angle may be shallow or sharp and range from less than or equal to 180 degrees to greater than or equal to 10 degrees. The connecting material 112 may in embodiments be formed with the attachment elements 40B and 45B, such as during an injection molding process, or may be later added after the attachment elements have been formed.

As shown in FIG. 6, connecting material 112 is positioned so that tube 16 may still be bonded to the attachment element, however in some embodiments, tube 16 may be attached only to the connecting material 112 and the attachment elements 40B and 45B do not physically contact tube 16. In these embodiment, the connecting material 112 is used to connect the attachment elements 40B and 45B to tube 16. FIG. 6 illustrates only one embodiment of using connecting material 112 with attachment elements 40B and 45B, other designs with different structures are contemplated within the scope of the present invention.

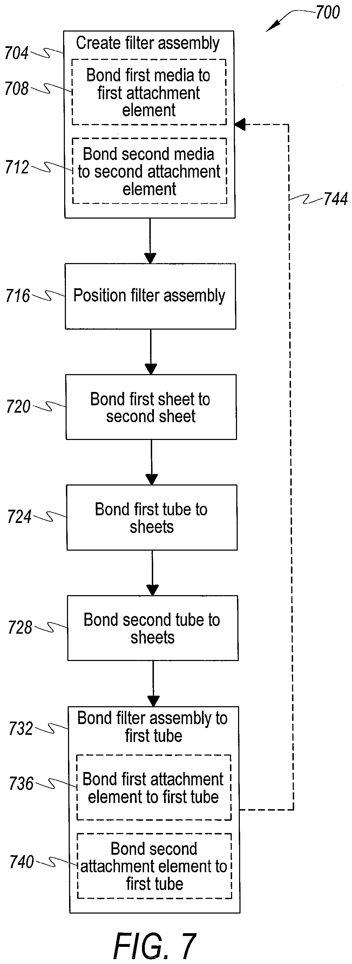

FIG. 7 shows a flow 700 that illustrates methods that may be performed to manufacture a filter. Although specific structures from FIGS 1-6 may be referred to below in describing the performance of methods, the present invention is not limited to these structures. For example, embodiments may include creating a filter assembly, which may be described below by referring to filter assembly 108 (FIGS. 1 and 2) but in other embodiments involve attachment elements different from attachment elements 40 and 45. The description below is provided merely for illustrative purposes, and flow chart 700 is not limited to manufacturing the structures shown in FIGS. 1-6, and may in other embodiments involve manufacturing structures that have features not described above with respect to FIGS. 1-6 above.

Flow 700 begins at step 704 where a filter assembly is created. In one embodiment, the filter assembly may be assembly 108 shown in FIGS. 1 and 2 above. As shown in FIG. 7, step 704 may involve one or more sub-steps. In embodiments, the sub-steps include bonding first media to a first attachment element at sub-step 708 and bonding second media to a second attachment element at sub-step 712. The bonding of media to attachment elements may involve attaching one or more layers of filter media to the attachment element. The bonding may involve one or more common bonding techniques, including without limitation, solvent welding, heat welding, high frequency welding (e.g., RF (radio frequency) welding or ultrasonic welding), and/or laser welding. The bonding techniques may depend on a number of different factors including the type of material from which the filter media and the attachment elements are made.

Flow 700 passes from step 704 to step 716, where the filter assembly created in step 704 is positioned prior to forming an outer housing of a filter, e.g., outer housing 120. In one embodiment, step 716 involves positioning the filter assembly between two sheets that will form the walls of an outer housing. In some embodiments the two sheets may be partially bonded together when step 716 is performed. In other embodiments, the filter assembly may be positioned adjacent one sheet before the second sheet is positioned on an opposite side of the filter assembly from the first sheet.

At step 720, a first sheet may be bonded to a second sheet to form an outer housing 120 of the filter. In embodiments, the first sheet and second sheet may be formed by flexible material such as polymeric sheets, e.g., sheets 10 and 11 described above. The bonding may involve one or more common bonding techniques, including without limitation, solvent welding, heat welding, high frequency welding (e.g., RF (radio frequency) welding or ultrasonic welding), and/or laser welding.

In some embodiments, step 720 may be performed multiple times. For example, a portion of the two flexible sheets may be partially bonded (to at least partially create a soft outer housing) before the filter assembly is positioned at step 716. In these embodiments, the sheets may be further bonded to form the final outer housing, after the filter assembly is positioned.

At step 724, a first tube is bonded to the first sheet and the second sheet of the outer housing. The first tube may be used in embodiments as an input or output for the filter, allowing fluid to enter or exit the outer housing. In embodiments, the first tube is positioned within a first port in the outer housing, such as port 104 (FIG. 2) before performing step 724 to bond the first tube to the first sheet and the second sheet. Step 724 may involve any of the bonding techniques described above.

At step 728, a second tube is bonded to the first sheet and the second sheet of the outer housing. The second tube may be used in embodiments as an input or output for the filter, depending upon for example whether the first tube is used as the input or the output. In embodiments, the second tube is positioned within a second port in the outer housing, such as port 100 (FIG. 2) before performing step 728 to bond the second tube to the first sheet and the second sheet. Step 728 may involve any of the bonding techniques described above.

At step 732, the filter assembly is bonded to the first tube. This step ensures that the filter assembly is secured and does not move around within the outer housing. This step may involve one or more sub-steps, such as sub-steps 736 and 740, where the first attachment element and the second attachment element are bonded to the first tube. In other embodiments, the sub-steps may involve bonding or otherwise attaching a structure of the filter assembly to the first tube. The first tube may be designed as the inlet for the filter or the outlet of the filter. Step 732 may involve any of the bonding techniques described above, and the specific bonding technique used will depend upon the materials of the filter assembly and the tube.

Although flow 700 has been described with steps listed in a particular order, the present invention is not limited thereto. In other embodiments, steps may be performed in different order, in parallel, or any different number of times, e.g., before and/or after another step. For example, is some embodiments, the step 732 (described above as bonding the filter assembly to the first tube) may be performed as part of step 704, namely creating the filter assembly (see line 744). In these embodiments, the sub-steps of bonding the first attachment element to the first tube (736) and bonding the second attachment element to the first tube (740) are performed as part of creating the filter assembly. As a result, when the filter assembly is created, it will include the first tube. This is merely one alternative example of different embodiments of flow 700.

Also, as indicated above, flow 700 includes some optional steps. However, those steps above that are not indicated as optional should not be considered as essential to the invention, but may be performed in some embodiments of the present invention and not in others.

The above has been described with respect to fibrous media formed into layers for filtering. It is understood, however, that the same attachment principles can apply to porous filter media.

## Claims

1. A filter (25) for filtering blood or blood components, the filter (25) comprising:
an inlet tube (15, 16);
an outlet tube (15, 16);
an outer housing (120) attached to the inlet tube (15) and the outlet tube (16), wherein the outer housing comprises a first flexible sheet and a second flexible sheet sealed to the first flexible sheet;
filter media (30, 32) within the outer housing (120);
a first attachment element (40, 40A, 40B) attached to one of the inlet (15, 16) or outlet (15, 16) tubes, **characterised in that** the first attachment element comprises:
a first wing (41, 41A) comprising a first tip; and
a second wing (42, 42A) comprising a second tip, wherein the first tip and second tip extend toward the first and second flexible sheets and contact the outer housing; and **in that**:
the filter media (30, 32) is attached to the first wing (41, 41A) and second wing (42, 42A) of the first attachment element (40, 40A, 40B).

2. The filter of claim 1, wherein said first attachment element attaches the filter media to the outlet tube.

3. The filter of claim 1, further comprising
a second attachment element (45, 45A, 45B) attached to the same one of the inlet and outlet tubes as the first attachment element and wherein the filter media is attached at a first end to the first and second attachment elements.

4. The filter of claim 3, wherein the filter media comprises layers and wherein the layers are sealed together at a second end opposite the first end of the filter media.

5. The filter of claim 3, wherein the one of the inlet and outlet tubes extends between the first and second attachment elements and between the filter media attached thereto.

6. The filter of claim 3, wherein the second attachment element comprises:
a third wing;
a fourth wing; and
wherein the filter media is attached to the third and fourth wings.

7. The filter of claim 6, wherein
the third wing comprises a third tip;
the fourth wing comprises a fourth tip; and
wherein the third and fourth tips extend toward the first and second flexible sheets and contact the outer housing.

8. The filter of claim 1, wherein the first attachment element is between the filter media and one of the inlet and outlet tubes.

9. The filter of claim 3, wherein the first attachment element is between the filter media and the one of the inlet and outlet tubes and wherein the second attachment element is between the filter media and the same one of the inlet and outlet tubes.

10. The filter of claim 1 wherein the first flexible sheet and the second flexible sheet are formed of soft thermoplastic sheets bonded together to form the outer housing.

11. A method of manufacturing a filter (25), the method comprising:
creating a filter assembly (25), wherein the creating comprises:
bonding a plurality of layers of first filter media (30) to a first and second wing (41, 42, 41A, 42A) of a first attachment element (40, 40A, 40B); and
bonding second filter media (32) to a second attachment element (45, 45A, 45B), wherein the second attachment element (45, 45A, 45B) is attached to the first attachment element (40, 40A, 40B);
positioning the filter assembly (25) between a first soft housing sheet (11) and a second soft housing sheet (10);
bonding the first soft housing sheet (11) to the second soft housing sheet (10) to create a soft outer housing (120) such that tips of the first and second wing contact the outer housing;
bonding a first tube (16) to the first soft housing sheet (11) and to the second soft housing sheet (10);
bonding a second tube (15) to the first soft housing sheet (11) and to the second soft housing sheet (10) ; and
bonding the first attachment element (40, 40A, 40B) to the first tube (16).

12. The method of claim 11, further comprising bonding the second attachment element to the first tube (16).

13. The method of claim 11, wherein the first tube (16) is positioned in a first port of the soft outer housing.

14. The method of claim 11, wherein the second tube (15) is positioned in a second port of the soft outer housing.

15. The method of claim 12, wherein the bonding the first attachment element to the first tube (16) is performed as part of the creating the filter assembly.

## Patentansprüche

1. Filter (25) zum Filtern von Blut oder Blutbestandteilen, wobei der Filter (25) Folgendes umfasst:
ein Einlassrohr (15, 16);
ein Auslassrohr (15, 16);
ein Außengehäuse (120), das am Einlassrohr (15) und am Auslassrohr (16) befestigt ist, wobei das Außengehäuse eine erste flexible Folie und eine zweite flexible Folie, die an die erste flexible Folie geschlossen ist, umfasst;
Filtermaterial (30, 32) innerhalb des Außengehäuses (120);
ein erstes Befestigungselement (40, 40A, 40B), das an einem von dem Einlass- (15, 16) oder Auslassrohr (15, 16) befestigt ist, **dadurch gekennzeichnet, dass** das erste Befestigungselement Folgendes umfasst:
einen ersten Flügel (41, 41A), umfassend eine erste Spitze; und
einen zweiten Flügel (42, 42A), umfassend eine zweite Spitze, wobei die erste Spitze und die zweite Spitze sich in Richtung der ersten und zweiten flexiblen Folie erstrecken und das Außengehäuse berühren; und dadurch, dass:
das Filtermaterial (30, 32) am ersten Flügel (41, 41A) und zweiten Flügel (42, 42A) des ersten Befestigungselements (40, 40A, 40B) befestigt ist.

2. Filter nach Anspruch 1, wobei das erste Befestigungselement das Filtermaterial am Auslassrohr befestigt.

3. Filter nach Anspruch 1, ferner umfassend
ein zweites Befestigungselement (45, 45A, 45B), das am selben von dem Einlass- und Auslassrohr befestigt ist wie das erste Befestigungselement, und wobei das Filtermaterial an einem ersten Ende am ersten und zweiten Befestigungselement befestigt ist.

4. Filter nach Anspruch 3, wobei das Filtermaterial Schichten umfasst, und wobei die Schichten an einem zweiten Ende gegenüber des ersten Endes des Filtermaterials zusammengeschlossen sind.

5. Filter nach Anspruch 3, wobei das eine aus Einlass- und Auslassrohr sich zwischen dem ersten und zweiten Befestigungselement und zwischen dem daran befestigten Filtermaterial erstreckt.

6. Filter nach Anspruch 3, wobei das zweite Befestigungselement Folgendes umfasst:
einen dritten Flügel;
einen vierten Flügel; und
wobei das Filtermaterial am dritten und vierten Flügel befestigt ist.

7. Filter nach Anspruch 6, wobei
der dritte Flügel eine dritte Spitze umfasst;
der vierte Flügel eine vierte Spitze umfasst; und
wobei die dritte und vierte Spitze sich in Richtung der ersten und zweiten flexiblen Folie erstrecken und das Außengehäuse berühren.

8. Filter nach Anspruch 1, wobei das erste Befestigungselement sich zwischen dem Filtermaterial und einem aus Einlass- und Auslassrohr befindet.

9. Filter nach Anspruch 3, wobei das erste Befestigungselement sich zwischen dem Filtermaterial und dem einen von dem Einlass- und Auslassrohr befindet, und wobei das zweite Befestigungselement sich zwischen dem Filtermaterial und demselben von dem Einlass- und Auslassrohr befindet.

10. Filter nach Anspruch 1, wobei die erste flexible Folie und die zweite flexible Folie aus weichen thermoplastischen Folien gebildet sind, die miteinander verbunden sind, um das Außengehäuse zu bilden.

11. Verfahren des Herstellens eines Filters (25), wobei das Verfahren Folgendes umfasst:
Schaffen einer Filtereinheit (25), wobei das Schaffen Folgendes umfasst:
Verbinden einer Vielzahl von Schichten eines ersten Filtermaterials (30) mit einem ersten und zweiten Flügel (41, 42, 41A, 42A) eines ersten Befestigungselements (40, 40A, 40B); und
Verbinden eines zweiten Filtermaterials (32) mit einem zweiten Befestigungselement (45, 45A, 45B), wobei das zweite Befestigungselement (45, 45A, 45B) am ersten Befestigungselement (40, 40A, 40B) befestigt ist;
Anordnen der Filtereinheit (25) zwischen einer ersten weichen Gehäusefolie (11) und einer zweiten weichen Gehäusefolie (10);
Verbinden der ersten weichen Gehäusefolie (11) mit der zweiten weichen Gehäusefolie (10), um ein weiches Außengehäuse (120) zu erzeugen, sodass Spitzen des ersten und zweiten Flügels das Außengehäuse berühren;
Verbinden eines ersten Rohrs (16) mit der ersten weichen Gehäusefolie (11) und der zweiten weichen Gehäusefolie (10);
Verbinden eines zweiten Rohrs (15) mit der ersten weichen Gehäusefolie (11) und der zweiten weichen Gehäusefolie (10); und
Verbinden des ersten Befestigungselements (40, 40A, 40B) mit dem ersten Rohr (16).

12. Verfahren nach Anspruch 11, ferner umfassend das Verbinden des zweiten Befestigungselements mit dem ersten Rohr (16).

13. Verfahren nach Anspruch 11, wobei das erste Rohr (16) in einer ersten Öffnung des weichen Außengehäuses angeordnet ist.

14. Verfahren nach Anspruch 11, wobei das zweite Rohr (15) in einer zweiten Öffnung des weichen Außengehäuses angeordnet ist.

15. Verfahren nach Anspruch 12, wobei das Verbinden des ersten Befestigungselements mit dem ersten Rohr (16) als Teil des Erzeugens der Filtereinheit durchgeführt wird.

## Revendications

1. Filtre (25) destiné à filtrer du sang ou des composants sanguins, le filtre (25) comprenant :
un tube d'admission (15, 16) ;
un tube d'évacuation (15, 16) ;
un boîtier externe (120) attaché au tube d'admission (15) et au tube d'évacuation (16) ; le boîtier externe comprenant une première feuille flexible et une deuxième feuille flexible scellée à la première feuille flexible ;
des milieux filtrants (30, 32) à l'intérieur du boîtier externe (120) ;
un premier élément d'attache (40, 40A, 40B) attaché à un des tubes d'admission (15, 16) ou d'évacuation (15, 16), **caractérisé en ce que** le premier élément d'attache comprend :
une première aile (41, 41A) comprenant un premier embout ; et
une deuxième aile (42, 42A) comprenant un deuxième embout, le premier embout et le deuxième embout s'étendant vers les première et deuxième feuilles flexibles et étant en contact avec le boîtier externe ; et **en ce que** :
les milieux filtrants (30, 32) sont attachés à la première aile (41, 41A) et à la deuxième aile (42, 42A) du premier élément d'attache (40, 40A, 40B).

2. Filtre selon la revendication 1, dans lequel ledit premier élément d'attache attache les milieux filtrants au tube d'évacuation.

3. Filtre selon la revendication 1, comprenant en outre
un deuxième élément d'attache (45, 45A, 45B) attaché au même tube des tubes d'admission et d'évacuation que le premier élément d'attache et les milieux filtrants étant attachés à une première extrémité des premier et deuxième éléments d'attache.

4. Filtre selon la revendication 3, dans lequel le milieu filtrant comprend des couches et dans lequel les couches sont scellées ensemble à une deuxième extrémité en face de la première extrémité des milieux filtrants.

5. Filtre selon la revendication 3, dans lequel l'un des tubes d'admission et d'évacuation s'étend entre les premier et deuxième éléments d'attache et entre les milieux filtrants attachés à ceux-ci.

6. Filtre selon la revendication 3, dans lequel le deuxième élément d'attache comprend :
une troisième aile ;
une quatrième aile ; et
les milieux filtrants étant attachés aux troisième et quatrième ailes.

7. Filtre selon la revendication 6, dans lequel
la troisième aile comprend un troisième embout ;
la quatrième aile comprend un quatrième embout ; et
le troisième et le quatrième embout s'étendant vers les première et deuxième feuilles flexibles et étant en contact avec le boîtier externe.

8. Filtre selon la revendication 1, dans lequel le premier élément d'attache se situe entre les milieux filtrants et le tube d'admission ou d'évacuation.

9. Filtre selon la revendication 3, dans lequel le premier élément d'attache se situe entre les milieux filtrants et ledit tube d'admission ou d'évacuation et dans lequel le deuxième élément d'attache se situe entre les milieux filtrants et le même tube des tubes d'admission et d'évacuation.

10. Filtre selon la revendication 1, dans lequel la première feuille flexible et la deuxième feuille flexible sont constituées de feuilles thermoplastiques souples liées ensemble pour former le boîtier externe.

11. Procédé de fabrication d'un filtre (25), le procédé comprenant :
la création d'un ensemble de filtres (25), la création comprenant :
la liaison d'une pluralité de couches d'un premier milieu filtrant (30) à une première et une deuxième aile (41, 42, 41A, 42A) d'un premier élément d'attache (40, 40A, 40B); et
la liaison d'un deuxième milieu filtrant (32) à un deuxième élément d'attache (45, 45A, 45B), le deuxième élément d'attache (45, 45A, 45B) étant attaché au premier élément d'attache (40, 40A, 40B) ;
le positionnement de l'ensemble de filtres (25) entre une première feuille de boîtier souple (11) et une deuxième feuille de boîtier souple (10) ;
la liaison de la première feuille de boîtier souple (11) à la deuxième feuille de boîtier souple (10) pour créer un boîtier externe souple (120) de sorte que des embouts des première et deuxième ailes soient en contact avec le boîtier externe ;
la liaison d'un premier tube (16) à la première feuille de boîtier souple (11) et à la deuxième feuille de boîtier souple (10) ;
la liaison d'un deuxième tube (15) à la première feuille de boîtier souple (11) et à la deuxième feuille de boîtier souple (10) ; et
la liaison du premier élément d'attache (40, 40A, 40B) au premier tube (16).

12. Procédé selon la revendication 11, comprenant en outre la liaison du deuxième élément d'attache au premier tube (16).

13. Procédé selon la revendication 11, dans lequel le premier tube (16) est positionné dans un premier orifice du boîtier externe souple.

14. Procédé selon la revendication 11, dans lequel le deuxième tube (15) est positionné dans un deuxième orifice du boîtier externe souple.

15. Procédé selon la revendication 12, dans lequel la liaison du premier élément d'attache au premier tube (16) est effectuée dans le cadre de la création de l'ensemble de filtres.
